# EUROPÄISCHE PATENTSCHRIFT

(11) **EP 3 414 547 B1**
(45) Veröffentlichungstag und Bekanntmachung des Hinweises auf die Patenterteilung: **02.07.2025**
(21) Anmeldenummer: 17707773.2
(22) Anmeldetag: 10.02.2017
(51) Int. Cl.: G01N 1/30, A61K 45/06, A61K 31/496

(54) **FIXATIVUM**
FIXATIV
FIXATIVE

(30) Priorität: 10.02.2016 DE 102016102346
(43) Veröffentlichungstag der Anmeldung: 19.12.2018
(73) Patentinhaber: Biosepar Gesellschaft für Medizin- und Labortechnik mbH, 84359 Simbach am Inn (DE)
(72) Erfinder: SZABADOS, Andreas, 82031 Grünwald (DE)
(74) Vertreter: von Bülow & Tamada
(86) Internationale Anmeldenummer: PCT/EP2017/053089
(87) Internationale Veröffentlichungsnummer: WO 2017/137616

(56) Entgegenhaltungen:
- EP-A1- 2 873 962
- EP-A2- 1 895 287
- DE-A1- 102012 101 896
- US-A1- 2010 209 930

## Beschreibung

Die vorliegende Erfindung betrifft ein Fixativum.

Diverse Fixativa ist beispielsweise aus der DE 10 2012 101 896 A1 bekannt. Eines der offenbarten Fixativa umfasst als Lösungsmittel Alkohol, in dem als Denaturierungsmittel ein Polyamin gelöst ist. Die DE 10 2012 101 896 A1 offenbart allerdings auch, dass bestimmte Alkohole, wie Ethanol als Lösungsmittel nachteilig sind, weil es das zu konservierende Gewebe dehydriert. Ein anderes der offenbarten Fixativa umfasst als Lösungsmittel Wasser, in dem als Denaturierungsmittel ein Polyamin gelöst ist. Insbesondere bei der Fixierung von Gewebe mit in Wasser gelösten Polyaminen als Denaturierungsmittel ist jedoch nachteilig, dass die zur Konservierung notwendige strukturelle Veränderung des zu konservierenden organischen Gewebes zu langsam abläuft und sich damit beispielsweise ungewünschte Artefakte bilden, oder der Fixiervorgang nicht alle Strukturen der Zellen und des Gewebes voll erfasst.

Die EP 2 873 962 A1 offenbart ein Fixativum gemäß dem Oberbegriff des geltenden Anspruchs 1.

Aufgabe der Erfindung ist es daher, die Wirkung des Denaturierungsmittels zu beschleunigen, ohne das zu konservierende Gewebe zu zerstören und so die Fixierung zu verbessern.

Gemäß einem Aspekt der Erfindung, umfasst ein Fixativum zur Konservierung von organischem Gewebe und Zellverbänden ein hydrophiles Denaturierungsmittel zum strukturellen Verändern der Moleküle des organischen Gewebes, ein lipophobes Lösungsmittel, in dem das Denaturierungsmittel gelöst ist und ein in dem Lösungsmittel gelöstes amphiphiles Infiltrationsmittel zum Lösen von Fetten an der Oberfläche des biologischen Gewebes, um ein Eindringen des Denaturierungsmittels in das biologische Gewebe zu beschleunigen.

Dem angegebenen Fixativum liegt die Überlegung zugrunde, dass ein Denaturierungsmittel zur Anwendung in einem Lösungsmittel gelöst verwendet wird und hydrophil wirkt. Das Denaturierungsmittel muss sich in dem Lösungsmittel lösen können, das Lösungsmittel ist daher nicht beliebig wählbar. Das Lösungsmittel darf das zu konservierende organische Gewebe dabei nicht verändern und sollte daher lipophob gewählt werden. Auf der anderen Seite verlangsamt Fett an den Zellwänden des zu konservierenden organischen Gewebes die Wirkung des Denaturierungsmittels, was die Wahrscheinlichkeit für osmotische Schäden am zu konservierenden organischen Gewebe erhöht, welche wiederum zu den zu vermeidenden Artefakten führen (Beispiele: Hämolytische Wirkung, strukturelle, biochemische und morphologische Zerstörung von Erythrozyten und anderen Zellen, Zellstrukturen, beispielsweise auch von Protozoen, Verschlechterung der immunologischen Eigenschaften). Das erfindungsgemäße Fixativum ist in Anspruch 1 definiert.

Für ein schnelles Einwirken des Denaturierungsmittels in das zu konservierende Gewebe wäre es daher wünschenswert, wenn das Fett an den Zellwänden möglichst schnell gelöst wird. Daher wird mit dem angegebenen Fixativum vorgeschlagen, in dem Lösungsmittel zusätzlich ein amphiphiles, also ein gleichzeitig lipophiles und hydrophiles, Infiltrationsmittel zu lösen, welches sich aufgrund seiner Hydrophilität im Lösungsmittel lösen lässt, und welches aufgrund seiner Lipophilität das Fett den Zellwänden des zu konservierenden biologischen Gewebes löst. Die Penetration des Denaturierungsmittels in das zu konservierende biologische Gewebe wird auf diese Weise beschleunigt und die Wirkung des Fixativums verbessert, um alle inter- und extrazellulären Bereiche beziehungsweise Bestandteile des zu konservierenden Gewebes zu erfassen.

Mit dem angegebenen Fixativum können selbst mit Polyamin, insbesondere auch mit Urotropin als Denaturierungsmittel zufriedenstellende Konservierungsergebnisse erreicht werden.

Als Lösungsmittel sollte bevorzugt Wasser verwendet werden, weil dies einerseits das zu konservierende Gewebe nicht angreift und andererseits sehr kostengünstig ist.

Das amphiphile Infiltrationsmittel sollte bevorzugt hygroskopische Eigenschaften aufweisen. Auf diese Weise wirkt das Infiltrationsmittel selbst fixierend und unterstützt das eigentliche Denaturierungsmittel bei der Konservierung, ohne dass ungünstige denaturierende und hygroskopische Eigenschaften zu Tage treten.

In dem amphiphilen Infiltatraionsmittel kann wenigstens ein kurzkettiges Alkanol mit maximal zehn Kohlenstoffeinheiten enthalten sein. Kurzkettige Alkohole haben sowohl eine polare OH-Gruppen als auch unpolare CH-Gruppen und wirken daher amphiphil. Das kurzkettige Alkanol kann aus Methanol, Ethanol, Isopropanol, Glyzerin und Butanol ausgewählt sein. Das angegebene Fixativum enthält erfindungsgemäß 80 bis 99,8 Gew. % des Lösungsmittels, 0,1 bis 10 Gew. % des Denaturierungsmittels und 0,1 bis 10 Gew. % des Infilitrationsmittels. Mit diesen Mischungsverhältnissen wird durch eine Lösung von lipophilen Membranstrukturen der Gewebszellen eine aktive Infiltration erzielt. Im eingangs genannten Fixativum wird durch Verwendung von amphiphilen Ethanol als Lösungsmittel lediglich eine passive Funktionshilfe erreicht, die bei vielen Gewebetypen nicht oder nur unvollkommen funktioniert.

Das Infiltrationsmittel enthölt erfindungsgemäß 0,1 bis 10 Gew % Methanol und/oder 0,1 bis 10 Gew % Ethanol und/oder 0,1 bis 10 Gew % Isopropanol und/oder 0,1 bis 10 Gew % Glyzerin und/oder 0,1 bis 10 Gew % Butanol. Insbesondere enthält das Fixativum erfindungsgemäß drei oder vier der genannten Alkohole und kann so ein ternäres oder quaternäres Gemisch sein.

Das Denaturierungsmittel kann ein Polyamin, insbesondere Urotropin (auch auch Hexamethylentetramin oder Methenamin genannt) enthalten. Das Fixativum kann ferner ein Säurungsmittel umfassen, das eingerichtet ist, Protonen abzuzgeben, die mit dem Denaturierungsmittel reagieren und so ein Aldehyd bilden, das dann wiederum die Konservierung des biologischen Gewebes beschleunigt. Mit dem Säurungsmittel kann der pH-Wert des Fixativums eingestellt werden. Wird beispielsweise Urotropin allein in Wasser als Lösungsmittel verwendet, dann ist der pH-Wert dieser Wasserlösung alkalisch und instabil. Für ein wirkungsvolles Fixativum sollte der pH-Wert auf einen stabilen Wert zwischen 2,5 und 10, vorzugsweise unterhalb von unter 8 eingestellt werden.

Das Säuerungsmittel zum Einstellen des pH-Wertes kann eine organische Säure, eine anorganische Säure, ein sauer reagierendes Salz oder eine Mischung davon sein.

Die organische Säure kann ausgewählt sein aus aliphatischen gesättigten und ungesättigten Monocarbonsäuren, Di- und Tricarbonsäuren, aromatischen Carbonsäuren, beispielsweise Salizylsäuren, Benzoesäuren, heterozyklischen Carbonsäuren oder Mischungen davon. Beispiele für in Frage kommende organische Säuren sind Ameisensäure, Essigsäure, Propionsäure, Milchsäure, Oxalsäure, Bernsteinsäure, Malonsäure, Glutarsäure, Weinsäure, Äpfelsäure, Zitronensäure, Sorbinsäure, Ascorbinsäure und deren Mischungen.

Die anorganische Säure kann ausgewählt sein aus Salzsäure, Schwefelsäure, schweflige Säure, Phosphorsäure, Salpetersäure, salpetrige Säure und Polythionsäuren und deren Salze, sowie deren Mischungen.

Das sauer reagierende Salz kann ausgewählt sein aus quartären Ammoniumverbindungen, Ammoniumchlorid und Mischungen davon.

Dem Fixativum können ferner bakterizid wirkende Substanzen (wie z.B. Natriumazid und quartäre Ammoniumverbindungen) beigemischt werden. Der Zusatz von Tensiden (anionischen und kationischen, nichtionischen und amphoteren Tensiden - hier auch quartäre Ammoniumverbindungen mit Tensidwirkung) verbessert die Fähigkeit der Infiltration und Penetration des in der Erfindung genannten Fixativums im physiologischen und nicht physiologischen Milieu. Als Konzentration kann 0,001 - 10,0% gewählt werden.

Die Lösungen mit den oben erwähnten Inhalten können ferner mit Duftstoffen und Farbstoff versehen.

Gegenüber den herkömmlichen Gewebe- und Zellfärbungen mit Formalin und anderen, bislang verwendeten konventionellen Fixiermittel mit vielen Nachteilen sind mit dem angegebenen Fixativum wenig, oder keine Artefakte zu verzeichnen. Viele Strukturen im Gewebe, in den Zellen und im subzellulären Bereich werden durch die verschiedenen nachfolgenden Färbeverfahren besser angefärbt. Durch die Verwendung des neu entwickelten Fixierkonzeptes werden auch die serologisch-immunologischen und molekularbiologischen Untersuchungen begünstigt im Sinne der verbesserten Möglichkeit der fachlichen und diagnostischen Beurteilung und Weiterverarbeitung.

Durch die Anfärbung des Fixiermittels erreicht man eine optische Differenzierung gegenüber anderen wässerigen Diagnostika und chemischen Lösungen. Dadurch kann die Sicherheit im Laboratorium erhöht werden. Die charakteristische Färbung, die mit geschützt werden soll: Pink-Farbe. Die Duftstoffe dienen ebenso der Sicherheit im Labor.

Durch das angegebene Fixativum findet keine nenneswerte Vernetzung von DNS und RNS und der Proteine statt, wie das bei den meisten bekannten Fixativa nach dem Stand der jetzt bekannten Technik gegeben ist. Das hat enorme Vorteile für die DNS/RNS-Untersuchungen, die molekular-biologische Gentechnologie, aber auch für alle anderen biotechnologischen Arbeiten und Untersuchungen, die mit Proteinstrukturen zu tun haben. Fixiermittel, die primäre, sekundäre, tertiäre oder quartäre Proteinstrukturen verändern und dies häufig sogar irreversibel, erfüllen nicht die modernen analytischen und präparativen Arbeitsbedingungen und deren Qualitätsmanagement. Durch die starke, zum Teil irreversible Proteindenaturierung wird nicht nur eine künstliche Barierre, Behinderung und Verzögerung des Fixationsvorganges erreicht. Es werden auch Artefakte produziert, die beispielsweise in der Diagnostik falsch positive oder auch falsch negative Ergebnisse begünstigen. Formalinhaltige Fixiermittel und deren Verwandte geben ein gutes Beispiel für diese Nachteile ab. Es muss aber hier auch auf die Nachteile der rein alkoholhaltigen Fixationsmittel verwiesen werden. Das angegebene Fixativum verbindet die Vorteile aller bisherigen Fixationsmittel nach dem Stand der Technik ohne deren Nachteile aufzuweisen.

Die oben beschriebenen Eigenschaften, Merkmale und Vorteile dieser Erfindung sowie Art und Weise, wie diese erreicht werden, werden klarer und deutlicher verständlich im Zusammenhang mit der folgenden Beschreibung der Ausführungsbeispiele, die im Zusammenhang mit der Zeichnung näher erläutert werden. Es zeigen:

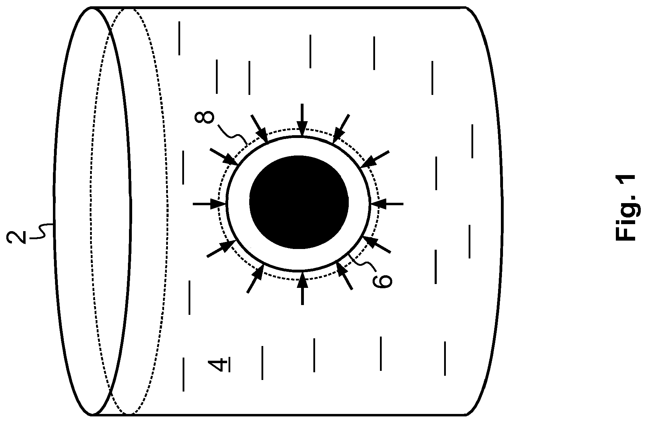

Fig. 1 eine schematische Darstellung eines zu konservierenden Gewebekörpers in einem Fixativum.

In den Figuren werden gleiche technische Elemente mit gleichen Bezugszeichen versehen und nur einmal beschrieben. Die Figuren sind rein schematisch und geben vor allem nicht die tatsächlichen geometrischen Verhältnisse wieder.

Es wird auf Fig. 1 Bezug genommen. Schematisch gezeigt ist ein Becherglas 2, in dem eine Fixierlösung 4 sowie ein mit der Fixierlösung 4 zu fixierendes Gewebe 6 aufgenommen ist. Das zu fixierende Gewebe 6 kann ein sehr fettreiches Organ eines Menschen sein, wie beispielsweise das Zentralnervensystem, die Leber, die Muskulatur, das Herz oder die Blutgefäße. Der reiche Fettgehalt des zu fixierenden Gewebes 6 ist in Fig. 1 durch eine gestrichelte Linie 8 um das zu fixierende Gewebe 6 angedeutet.

Die Fixierlösung 4 enthält im vorliegenden Ausführungsbeispiel 98% eines Lösungsmittels in Form von Wasser, 1% eines Denaturierungsmittels in Form von Urotropin und 1% eines noch zu beschreibenden Infiltrationsmittels.

Beispielsweise aus der DE 10 2012 101 896 A1 ist die konservierende Wirkung von Urotropin bekannt. Allerdings läuft der Konservierungsvorgang nur sehr langsam ab und insbesondere ein hoher Fettanteil 8 in dem zu konservierenden organischem Gewebe 6 verlängert den Konservierungsvorgang deutlich.

Aus diesem Grund ist in dem Lösungsmittel der Fixierlösung 4 zusätzlich das Infiltrationsmittel gelöst. Seine Aufgabe ist es, den Fettanteil 8 des zu fixierenden Gewebes zu lösen und so die Infiltration des Dentaturierungsmittels zu beschleunigen. Damit das Infiltrationsmittel in der Fixierlösung 4 gelöst werden kann, muss es zusätzlich hydrophil gewählt sein, weshalb für das Infiltrationsmittel ausschließlich amphiphile Stoffe in Frage kommen.

In der vorliegenden Ausführung wurde für das Infiltrationsmittel ein quaternäres Gemisch aus Methanol, Ethanol, Isopropanol und Glyzerin zu je gleichen Anteilen gewählt. Durch Hinzugeben dieses Infiltrationsmittels zu der Fixierlösung 4 konnte die Zeitdauer des gesamten Fixier- und Konservierungsvorgang deutlich reduziert werden.

Zwar ist die Verwendung eines amphiphilen Mittels in Form von Alkohol in der Fixierlösung bereits aus der DE 10 2012 101 896 A1 bekannt, allerdings wird der Alkohol dort als Lösungsmittel selbst verwendet. Der dort angegebene Alkohol begünstigt, vermischt mit Urotropin als ein Mittel mit stark polarer und hydrophiler Eigenschaft, das passive Eindringen ins biologische Gewebe.

Die Eigenschaft der zuvor als Infiltrationsmittel angegebenen Alkohole aufgelöst in Wasser erweitert einerseits das Eindringen des Fixiermittels Urotropin in einer deutlich verbesserten Weise, nämlich durch Erweichung/Auflösung - und Öffnung - der lipophilen Membranstrukturen, andererseits wirken diese Alkohole selbst in den vorgeschlagenen Konzentrationen und Mischungen auch als ideale Fixiermittel - ohne die ungünstigen denaturierenden und hygroskopischen Eigenschaften der reinen Alkoholfixierungen zu besitzen, wenn der Alkohol als Lösungsmittel selbst verwendet wird.

Zudem sind die Fixiereigenschaften der zusammen mit Urotropin in Wasser gelösten Alkohole in den angegebenen Konzentrationen nicht nur einzeln, sondern auch zusammen kumulativ gegeben, wodurch eine deutliche Steigerung der fixativen Qualität und Potenz bei allen Gewebetypen unabhängig vom Fettgehalt gegeben ist.

Zusammenfassend lässt sich die angegebene Fixierlösung wie folgt beurteilen:
Amphiphile Stoffe, wie die beispielhaft angegebenen Alkohole werden nicht als das Denaturierungsmittel tragendes Lösungsmittel sondern als Hilfsmittel verwendet. Die für das angegebene Fixativum angegebenen Stoffgruppen wirken in den angegebenen Konzentrationen und im vorgeschlagenen Mischungverhältnis in idealer Weise fixierend im physiologischen Milieu wie auch bei verfettetem oder fettreichem Gewebe.

Das angegebene Fixtativim eliminiert die bisherigen Nachteile der einzelnen fixativ wirkenden amphiphilen Stoffe, weil diese Nachteile durch das lösen in einem hydrophoben Lösungsmittel nicht mehr zum Tragen kommen.

Die Infiltration des Denaturierungsmittels wird aktiv durch Lösung von lipophilen Membranstrukturen der Gewebszellen erzielt. Bei der Verwendung amphiphiler Stoffe als Lösungsmittel konnte lediglich eine passive Funktionshilfe erzielt werden, die bei vielen Gewebetypen nicht oder nur unvollkommen funktioniert.

## Patentansprüche

1. Fixativum zur Konservierung von organischem Gewebe und Zellverbünden, umfassend:
- ein hydrophiles Polyamin enthaltendes Denaturierungsmittel zum strukturellen Verändern der Moleküle des organischen Gewebes,
- ein lipophobes Lösungsmittel, in dem das Denaturierungsmittel gelöst ist, und
- ein in dem Lösungsmittel gelöstes amphiphiles Infiltrationsmittel zum Lösen von Fetten an der Oberfläche des biologischen Gewebes, um ein Eindringen des Denaturierungsmittels in das biologische Gewebe zu beschleunigen,
**gekennzeichnet durch**
- 80 bis 99,8 Gew. % des Lösungsmittels,
- 0,1 bis 10 Gew. % des Denaturierungsmittels, und
- 0,1 bis 10 Gew. % des Infilitrationsmittels, wobei das amphiphile Infiltatraionsmittel Methanol oder Ethanol enthält,
und das Infiltrationsmittel mindestens drei, vorzugsweise vier der folgenden Alkohole enthält:
- 0,1 bis 10 Gew % Methanol, und/oder
- 0,1 bis 10 Gew % Ethanol, und/oder
- 0,1 bis 10 Gew % Isopropanol, und/oder
- 0,1 bis 10 Gew % Glyzerin, und/oder
- 0,1 bis 10 Gew % Butanol.

2. Fixativum nach Anspruch 1, wobei das Lösungsmittel Wasser enthält.

3. Fixativum nach Anspruch 1 oder 2, wobei das amphiphile Infiltrationsmittel hygroskopische Eigenschaften aufweist.

4. Fixativum nach einem der vorstehenden Ansprüche, wobei das Polyamin Urotropin ist.

5. Fixativum nach einem der vorstehenden Ansprüche, umfassend ein Säurungsmittel, das eingerichtet ist, Protonen abzugeben, die mit dem Denaturierungsmittel reagieren.

6. Fixativum nach Anspruch 5, wobei das Säurungsmittel eine organische Säure, eine anorganische Säure, ein sauer reagierendes Salz oder eine Mischung davon sein.

7. Fixativum nach einem der vorstehenden Ansprüche, ferner umfassend eine bakterizid wirkende Substanz.

## Claims

1. Fixative for the preservation of organic tissue and cell associations, comprising:
- a denaturing agent containing a hydrophilic polyamine for structurally altering the molecules of the organic tissue,
- a lipophobic solvent in which the denaturing agent is dissolved, and
- an amphiphilic infiltration agent dissolved in the solvent for dissolving fats on the surface of the biological tissue to accelerate the penetration of the denaturing agent into the biological tissue,
**characterized by**
- 80 to 99.8 wt.% of the solvent,
- 0.1 to 10 wt.% of the denaturing agent, and
- 0.1 to 10 wt.% of the infiltration agent, wherein the amphiphilic infiltration agent contains methanol or ethanol,
and the infiltration agent contains at least three, preferably four, of the following alcohols:
- 0.1 to 10 wt.% methanol; and/or
- 0.1 to 10 wt.% ethanol; and/or
- 0.1 to 10 wt.% isopropanol; and/or
- 0.1 to 10 wt.% glycerol; and/or
- 0.1 to 10 wt.% butanol.

2. Fixative according to claim 1, wherein the solvent contains water.

3. Fixative according to claim 1 or 2, wherein the amphiphilic infiltration agent exhibits hygroscopic properties.

4. Fixative according to any of the preceding claims, wherein the polyamine is hexamethylenetetramine.

5. Fixative according to any of the preceding claims, further comprising an acidifying agent configured to donate protons that react with the denaturing agent.

6. Fixative according to claim 5, wherein the acidifying agent is an organic acid, an inorganic acid, an acid-reactive salt, or a mixture thereof.

7. Fixative according to any of the preceding claims, further comprising a bactericidal substance.

## Revendications

1. Fixateur pour la conservation de tissus organiques et d'agrégats cellulaires, comprenant :
- un agent de dénaturation contenant une polyamine hydrophile destiné à modifier structurellement les molécules du tissu organique,
- un solvant lipophobe dans lequel l'agent de dénaturation est dissous, et
- un agent d'infiltration amphiphile dissous dans le solvant, destiné à dissoudre les graisses présentes à la surface du tissu biologique afin d'accélérer la pénétration de l'agent de dénaturation dans le tissu biologique,
**caractérisé par**
- 80 à 99,8 % en poids du solvant,
- 0,1 à 10 % en poids de l'agent de dénaturation, et
- 0,1 à 10 % en poids de l'agent d'infiltration, l'agent d'infiltration amphiphile contenant du méthanol ou de l'éthanol,
et l'agent d'infiltration contenant au moins trois, de préférence quatre, des alcools suivants :
- 0,1 à 10 % en poids de méthanol ; et/ou
- 0,1 à 10 % en poids d'éthanol ; et/ou
- 0,1 à 10 % en poids d'isopropanol ; et/ou
- 0,1 à 10 % en poids de glycérine ; et/ou
- 0,1 à 10 % en poids de butanol.

2. Fixateur selon la revendication 1, dans lequel le solvant contient de l'eau.

3. Fixateur selon la revendication 1 ou 2, dans lequel l'agent d'infiltration amphiphile présente des propriétés hygroscopiques.

4. Fixateur selon l'une quelconque des revendications précédentes, dans lequel la polyamine est l'urotropine.

5. Fixateur selon l'une quelconque des revendications précédentes, comprenant en outre un agent acidifiant conçu pour libérer des protons réagissant avec l'agent de dénaturation.

6. Fixateur selon la revendication 5, dans lequel l'agent acidifiant est un acide organique, un acide inorganique, un sel à réaction acide ou un mélange de ceux-ci.

7. Fixateur selon l'une quelconque des revendications précédentes, comprenant en outre une substance à effet bactéricide.
